# EUROPEAN PATENT SPECIFICATION

(11) **EP 3 897 972 B1**
(45) Date of publication and mention of the grant of the patent: **02.10.2024**
(21) Application number: 19818147.1
(22) Date of filing: 17.12.2019
(51) Int. Cl.: B01J 20/289, B01J 20/30, B01D 15/08, C07K 1/16

(54) **LIGAND LINKER SUBSTRATE**
LIGAND-LINKERSUBSTRAT
SUBSTRAT À COUPLEUR DE LIGANDS

(30) Priority: 17.12.2018 EP 18213244
(43) Date of publication of application: 27.10.2021
(60) Divisional application: 22194180.0
(73) Proprietor: CHRETO ApS, 3500 Værløse (DK); 3M Innovative Properties Company, Saint Paul, MN 55133-3427 (US)
(72) Inventor: KYHSE-ANDERSEN, Jan, 3500 Værløse (DK); WINTHER, Lars, 2765 Smørum (DK); RASMUSSEN, Jerald, Saint Paul, MN 55133 (US)
(74) Representative: Inspicos P/S
(86) International application number: PCT/EP2019/085711
(87) International publication number: WO 2020/127311

(56) References cited:
- EP-B1- 2 220 107
- US-B2- 8 795 782
- WANG Y. ET AL: "Identification of Affinity Ligands for Protein Purification from Synthetic Chemical Combinatorial Libraries", BIOTECHNOLOGY PROGRESS, vol. 18, no. 3, 7 June 2002 (2002-06-07), pages 524 - 529, XP055956399, ISSN: 8756-7938, DOI: 10.1021/bp020033d
- JIANG LIUWEI ET AL: "Biotin-functionalized poly(ethylene terephthalate) capillary-channeled polymer fibers as HPLC stationary phase for affinity chromatography", ANALYTICAL AND BIOANALYTICAL CHEMISTRY, SPRINGER, DE, vol. 407, no. 3, 20 November 2014 (2014-11-20), pages 939 - 951, XP035434155, ISSN: 1618-2642, [retrieved on 20141120], DOI: 10.1007/S00216-014-8235-4
- SIGMA-ALDRICH: "Biotin-Agarose saline suspension | Sigma-Aldrich", 1 March 2018 (2018-03-01), XP055668283, Retrieved from the Internet <URL:https://www.sigmaaldrich.com/catalog/product/sigma/b6885?lang=en&region=NL> [retrieved on 20200213]
- THERMO SCIENTIFIC: "Thermo Scientific Avidin-Biotin Technical Handbook-", 23 March 2009 (2009-03-23), XP055668268, Retrieved from the Internet <URL:http://assets.thermofisher.com/TFS-Assets/LSG/brochures/1601675-Avidin-Biotin-Handbook.pdf> [retrieved on 20200213]

## Description

### FIELD OF THE INVENTION

The present invention relates to receptor-ligand kinetics, increased binding rates and increased dynamic binding capacity of receptor of interest to a functionalised substrate.

The functionalized substrates, methods of preparing said ligand-linker functionalized substrates and the linker length optimisation thereof. More specifically, the ligand linker functionalized substrates include a solid substrate, which has been modified to provide binding ligand groups covalently bound via a linker length, used to increase the binding rate and the dynamic binding capacity of receptor compound of interest to substrate.

### BACKGROUND OF THE INVENTION

Isolation and purification of target molecules, such as biomacromolecules, are important for therapeutic purposes and in biomedical research. The general industrial purification process often includes a number of unit operations, like extraction, filtration, precipitation, as well as anion- and cation-exchange chromatography, and affinity purification.

Most current capture or affinity purification chromatography is done via conventional resin based column techniques. Porous beaded chromatography resins have high surface area to mass ratio bearing interactive affinity ligand groups, and have therefore been used in protein affinity purification processes. These techniques are time consuming and provide severe bottlenecking issues in downstream purification, due to binding rates dependent on mass transport by diffusion. This also limits the chromatography techniques applicability in high throughput screening and for rapid downstream processing.

Filter, membrane, nano-fibre and nano-particles -based technologies, especially in disposable format, are becoming increasingly important in biopharmaceutical and vaccine manufacturing processes. Membranes have been used in passive, molecular size-based separation and in active filtration.

Functionalized membranes having enhanced process flow properties (including functional polymer-bearing membranes) have typically suffered from relatively low biomaterial binding capacities due to low surface area to mass ratio, which in general have limited their use in certain large-scale purification processes.

However, membranes and filters have high productivity potential (product mass/volume/time) due to the possible high process stream velocities and efficient mass transport by convection. Today membranes and filters are mainly used in polishing processes to remove impurities to very low levels in product streams. These materials, membranes, filters and porous bead resins, could be improved further. Also, bead resin-based materials could be more economical applicable by optimising the dynamic binding kinetic between ligand and receptors. The resulting membrane, filter or bead processes will be even faster and more efficient and make it possible to have downstream purification unit operations with smaller foot print, higher capacity and provide less waste water and consumption of process buffer fluids.

Standard ligand-functionalized substrates, such as ligand-functionalized membranes, are widely used for the separation and affinity purification of various target molecules. For example, ligand-functionalized membranes can be used to purify or separate a target molecule based on an affinity interaction, or based on the formation of a covalent bond.

US 5,451,453 discloses substrates having azlactone-functional surfaces, adduct substrates, and methods of preparing both.

US 9,616,394 B2 discloses guanidinyl ligand-functionalized polymers, methods of making the same, and substrates bearing a grafted coating of the ligand-functional polymers.

US 9,650,470 B2 discloses ligand functionalized substrates, methods of making ligand functionalized substrates, and methods of using functionalized substrates.

US 9,958,364 B2 discloses ligand functionalized substrates with enhanced binding capacity.

US 9,758,547 B2 discloses ligand functionalized substrates, methods of making ligand functionalized substrates, and methods of using functionalized substrates.

EP 2220107 B1 discloses a process for purification of a target biomolecule, comprising the steps: (a) contacting (i) a target biomolecule, (ii) a dual affinity polypeptide (DAP), and (iii) a solid substrate comprising a catching ligand; and (b) recovering the target biomolecule by elution, wherein the target biomolecule and the dual affinity polypeptide are contacted in solution before the mixture is contacting the solid substrate.

WO 2010/128033 A2 discloses a process for purification of a target molecule, comprising the steps: (a) contacting a target molecule, and a population of target binding polypeptides (TBP), in solution for a sufficient time to allow complex formation; and (b) isolating the target from the complex from (a) by subsequent purification steps, wherein (i) the target binding polypeptides have at least two binding functionalities; a first binding functionality towards the target and a second binding functionality towards a catching ligand comprised in a solid substrate; and (ii) the first binding functionality comprises at least two binding sites for the target, and the target comprises at least two binding sites for the TBP.

The dual affinity polypeptide (DAP) described in EP 2220107 B1 have the same functionality as the target binding polypeptides (TBP) described in WO 2010/128033 A2 and the two terms DAP and TBP is used herein interchangeably to describe the same compound. Wang Y.

ET AL: "Identification of Affinity Ligands for Protein Purification from Synthetic Chemical Combinatorial Libraries",Biotechnology Progress, vol. 18, no. 3, 7 June 2002 (2002-06-07), pages 524-529, DOI: 10.1021/ bp020033d describes a ligand-functionalized substrate comprising an Ultralink resin support to which a biotin group is coupled via a linker.

While a number of ligand-functionalized substrates exist, there is a need in the art for more optimal ligand-linker functionalized substrates that can operated at a high binding rate and with high dynamic binding capacity to provide high product throughput - and thereby high productivity to reduce manufacturing equipment investment, operating cost and environmental foot print.

### OBJECT OF THE INVENTION

It is an object of embodiments of the invention to provide a ligand-linker functionalized substrate that allows a fast binding rate to the receptor (compound of interest) without compromising a high dynamic binding capacity.

### SUMMARY OF THE INVENTION

It has been found by the present inventors that by providing a ligand-functionalized substrate wherein the ligand is bound to the substrate via a linker of a specific length and preferably a non-bendable structure, a considerably increased dynamic binding capacity as well binding rate between the ligand and a receptor of interest may be obtained.

So, in a first aspect the present invention relates to a ligand-functionalized substrate as defined in claim 1 comprising:
a. A substrate having carboxylic (-COOH), hydroxy (-OH), thio (-SH), amino groups (-NH2), C-C double bonds (-ene) or C-C triple bonds (-yne) at the surface thereof;
b. Covalently bound to said carboxylic (-COOH), hydroxy (-OH), thio (-SH), amino groups (-NH2), C-C double bonds (-ene) or C-C triple bonds (-yne) of said substrate a linker, such as a semi-flexible linker, having a linker length of 10-25, such as 17-22 bonds, wherein bonds are C-C, C-N, C-N(H), C-C(O) and/or C-O; and
c. A ligand-functional group bound to the surface of the substrate via said linker.

In a second aspect the present invention relates to a method for the preparation of the ligand-functionalized substrate according to claim 1, comprising (a) activating the substrate chemically or by introducing a radical, and (b) coupling the ligand to the substrate directly via the linker or by radical co-polymerization using two or more acrylic monomers, of which at least one contains a ligand.

In a third aspect the present invention relates to a use of a ligand-functionalized substrate according to the invention for capturing or immobilizing a receptor of interest.

### LEGENDS TO THE FIGURE

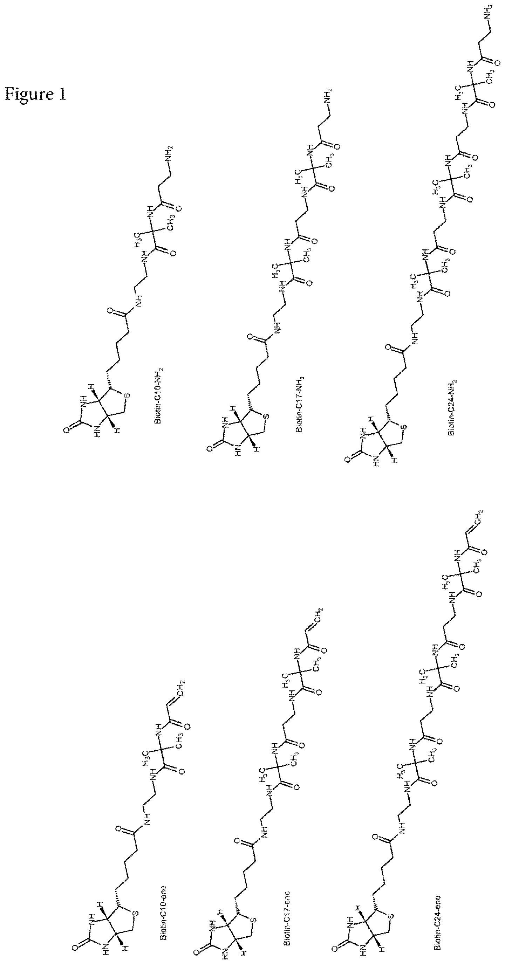

Fig. 1 illustrates the structure and nomenclature of the "shorter", "medium" and "long" linker biotin affinity ligands with terminal amino -NH2 (series A), Biotin-C10-NH2, Biotin-C17-NH2 and Biotin-C24-NH2 and terminal -ene (series B) Biotin-C10-ene, Biotin-C17-ene, Biotin-C24-ene..
Fig. 2 illustrates the determination of the different length of the linkers.
Fig, 3 illustrates the composition of the Biotin-linker (ethylendiamnie EDA, alpha-methyl alanine and beta-alanine)
Fig. 4 illustrates the synthesis of the Biotin-C10-ene
Fig. 5 illustrates the synthesis of the Biotin-C10-NH2

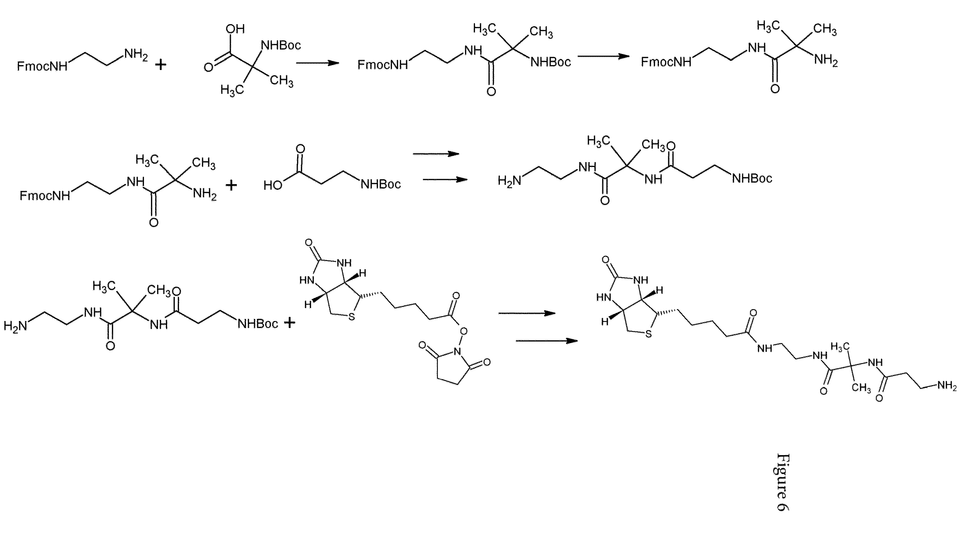

Fig. 6 illustrates the alternative synthesis route for Biotin-C10-NH2
Fig. 7 illustrates the synthesis of Biotin-C17-ene.
Fig. 8 illustrates the synthesis of Biotin-C17-NH2
Fig. 9 illustrates the synthesis of Biotin-C24-ene
Fig.10 illustrates the synthesis of Biotin-C24-NH2
Figure 11. Measurement of dynamic binding capacity using Hanes plot - linearized form of the Langmuir isotherm - for Biotin-C17 functionalized solid substrate. The equation of the trendline is y=13.08x + 3,2307. The slope of the trendline equals the maximum binding capacity and is 13,08 mg DAP/TBP in 0,25 mL solid substrate. This corresponds to a DBC of 52.3 mg/mL.

### DETAILED DISCLOSURE OF THE INVENTION

### Definitions

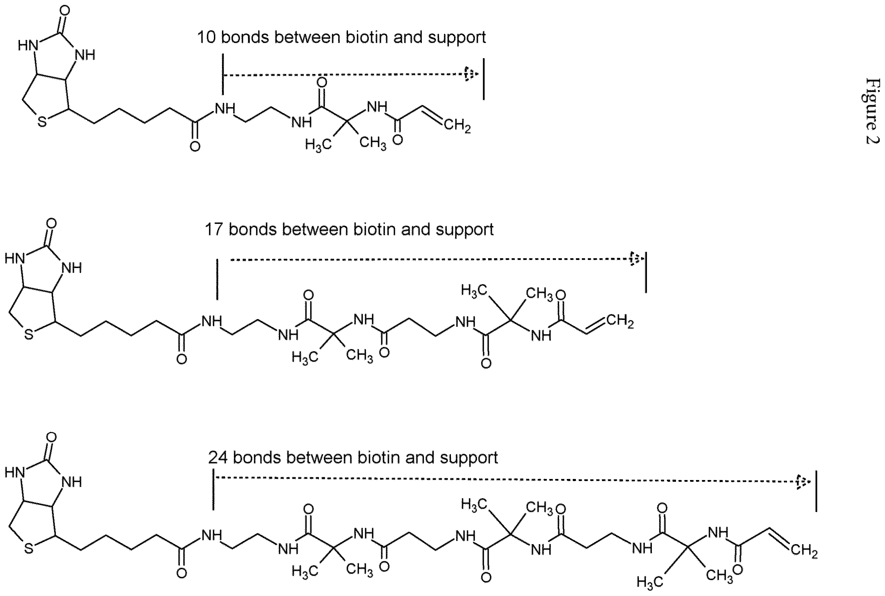

In the present context the length of the linker is defined by the number of bonds, wherein bonds are C-C, C-N, C-N(H), C-C(O) and/or C-O bonds. Fig. 2 further illustrates how the number of linker bonds is determined. It is noted that biotin by nature contains a lipophilic linker. The linker used in accordance with the present invention contains amide and partly sterically hindered groups, which prevents the linker from being fully flexible.

It is to be understood that a linker refers to a chain of at least 6 N, C, S or O atoms linking the substrate and ligand-functional group of the invention. Typically, the linker is a linear sequence of at least 6 N, O or C atoms, wherein the length of the linker is defined by the total amount of bonds in the linear sequence of atoms (see fig 2). Bonds may include C-C, C-N, C-N(H), C-C(O) and/or C-O.

It should be understood that the practical length of the linker depends on the actual lengths of the individual chemical bonds, the surrounding functionalities, the angles, the rotational flexibility and the conformation in the solvent. For example, the average C-C, C-O and C(O)-N(H) bond are about 1.5, 1.4 and 1.3 Å, respectively. The C-O bond can partly rotate, whereas the amide bond is not as free to rotate.

The linker length is defined as the number of chemical bonds in the linker. That is from the ligand functional group, such as a biotin to the substrate. It is noted that the terminal bond to the substrate is also counted. Accordingly, when counting the length of a linker as shown herein having a terminal -ene, the bond from the terminal carbon atom to the substrate should also be counted. If the linker as shown herein has a terminal amino groups (-NH2), the bond to this amino groups will be counted as the last bond. One should be aware, that the biotin has naturally C5 linker between the ring structure, the ureido and tetrahydrothiophene groups, and the carboxyl group.

The biotin-binding domain in avidin/streptavidin molecules are buried about 9 A below the protein surface and hence. The presence of bulky groups in the vicinity of the biotin-binding pocket may create steric hindrances and reduce the binding efficiency.

It should be understood that the functionality and the length of the linker is associated with the dynamic binding capacity and the binding rates of the DAP/TBP molecules to the surface.

As used herein a "dual affinity polypeptide" or "DAP" used interchangeably with "target binding polypeptides" or "TBP" refers to a recombinant protein comprising at least one binding domain capable of binding to the target biomolecule with the desired binding specificity as described herein, as well as at least one binding domain capable of binding to the ligand-functionalized substrate according to the invention. Suitable DAP molecules are described in WO2009062942 and suitable TBP molecules is described in WO 2010/128033 A2 includes in a preferred embodiment a streptavidin monomer fused in a fusion protein to four protein A binding domains.

### Specific embodiments of the invention

The present invention related in a broad aspect to a ligand-functionalized substrate comprising: a) A substrate having carboxylic (-COOH), hydroxy (-OH), thio (-SH), amino groups (-NH2) C-C double bonds (-ene) or C-C triple bonds (-yne) at the surface thereof; b) Covalently bound to said carboxylic (-COOH), hydroxy (-OH), thio (-SH), amino groups (-NH2), C-C double bonds (-ene) or C-C triple bonds (-yne) of said substrate a linker having a linker length of 10-25, such as 17 -22 bonds, wherein bonds are C-C, C-N, C-N(H), C-C(O) and/or C-O; and c) A ligand-functional group bound to the surface of the substrate via said linker.

According to the invention, the ligand-functionalized substrate according to the invention the substrate is a solid substrate, such as in the form of sheets, membranes, beads, saddles or granulates. With both smooth surfaces and large inner surfaces due to porosity.

In an embodiment of the ligand-functionalized substrate according to the invention the substrate is a solid substrate which is grafted by radical initiated vinyl polymerization.

In an embodiment of the ligand-functionalized substrate according to the invention the substrate is selected from the group consisting of agarose, kieselguhr, silica gel, cellulose, cellulose ethers, carboxymethyl cellulose, degenerated cellulose, agarose or paper based membranes, nitrocellulose, nitrocellulose mixed esters, silicas, and controlled pore glasses, polyamides, poly sulfone ether, polyvinylalcohols, polycarbonate, polyurethane, polyethersulfone, polysulfone, polyethylene terephthalate, polyvinylidene fluoride, polystyrene or polypropylene, polyethylene and co-block polymers, blends and combinations hereof. In an embodiment the substrate is in the form of particles, films, woven or nonwoven webs, sponges, fibres, sheets, beads, filter, or a membrane. The substrate used according to the invention is usually porous for increased surface area and optimal liquid flow properties and can be both organic and inorganic, including polymeric.

The synthesis methods of stepwise elongating peptides by stepwise coupling protected amino acids or other multifunctional building blocks together is well-described from for example classical Merrifield solid phase peptide synthesis or in solution. The various chemo selective and orthogonal protection groups include Boc, Fmoc, Benzyl or t-But among others. The amide formation is done using for example carbodiimides, efficient active ester, HATU/HOAt or similar reactions. A summary of SPPS reagents and methods can be found in for example Merrifield, R. B. In Peptides: "Synthesis, Structures and Applications"; (Gutte, B., Ed.); Academic Press: San Diego, CA, 1995; pp 93-168 or in Fernando Albericio (ed.): "Solid-Phase Synthesis: A Practical Guide", CRC Press 2000. and in more general for conjugation and cross-linking in Shan S. Wong, "Chemistry of Protein Conjugation and Cross-Linking", CRC Press, 1991.

Also, the use of 2-Vinyl-4,4-dimethyl azlactone (VDMA) and derivatives for elongating and grafting linkers are well known. For example described in Jamie M. Messman et al Macromolecules 2009, 42, 3933-3941, US2016231208, and other references cited herein.

In the ligand-functionalized substrate according to the invention the ligand functional group is biotin.

The present inventors have surprisingly found that the length and nature of the linker has a distinct influence on both the dynamic binding capacity (DBC) and binding rate of the resulting use thereof for binding a receptor molecule like e.g. dual affinity polypeptide (DAP) or target binding protein (TBP) and efficiently removing it from solution, which is a requirement for the purification of a target biomolecule. The linker should preferably be semi flexible in water at pH 4, to allow a stretched configuration and thereby higher binding efficiency. The linker between the substrate and the ligand-functional group, such as biotin, should be extended in water, but not be completely flexible or free to rotate, or bend in all directions.

Further, the inventors have found that the optimal length for reaching into a ligand functional group being a biotin binding pocket require guidance to the optimal dynamic binding efficiency. The various linker optimizations described in the prior art are typically based on steady state equilibrium, static binding conditions or from experiments with small molecules or from anion- and cation-exchange chromatography.

In an embodiment of the use according to the invention the use is for purification of a target biomolecule, comprising the steps:
a) contacting (i) a target biomolecule, (ii) a dual affinity polypeptide (DAP), and (iii) a ligand-functionalized substrate according to the invention, and (b) recovering the target biomolecule by elution, wherein the target biomolecule and the dual affinity polypeptide are contacted in solution before the mixture is contacting the ligand-functionalized substrate.

In an embodiment of the use according to the invention the use is for purification of a target biomolecule, comprising the steps:
a) contacting a target molecule, and a population of target binding polypeptides (TBP) or dual affinity polypeptide (DAP), in solution for a sufficient time to allow complex formation; and (b) isolating the target from the complex from (a) by subsequent purification steps, wherein (i) the target binding polypeptides have at least two binding functionalities; a first binding functionality towards the target and a second binding functionality towards a catching ligand comprised in a solid substrate; and (ii) the first binding functionality comprises at least two binding sites for the target, and the target comprises at least two binding sites for the TBP/ DAP.

In some embodiments, the method comprises a step of washing away impurities, such as host cell proteins (HCP), DNA, viruses, selectively immobilizing or removing the dual affinity polypeptide from target product solution.

In an embodiment thereof the target biomolecule is an antibody.

It is to be understood that the term antibody as used herein may refer to any immunoglobulin molecule, such as an immunoglobulin molecule with binding affinity to Protein A, such as any immunoglobulin molecule comprising an Fc region, or a Fab region, such as from IgG of the human VH3 gene family.

### EXAMPLES

The following examples illustrate the present invention. In summary, a number of linkers according to the invention was prepared and the dynamic binding capacities and binding rates were measured.

In more detail, biotin was functionalized with an amin. By stepwise synthesis using VDMA, the linkers were constructed with either an acrylic or amine end group. The same method was used to synthesis longer versions of the linker. In an alternative synthesis route, the linkers of various lengths were synthesized using step wise amide couplings, similar to SPPS methods.

The various linkers were coupled or grafted to the substrates and tested for dynamic binding capacity and binding rates. The measured dynamic capacity and rates depended on the linker lengths.

Analytical methods Flash column chromatography was performed on a Teledyne ISCO, Combi Flash Rf+ Lumen using RediSepRf alumina basic or SiliCycle SiliaSep silica columns.

Analytical HPLC-MS was performed using an Agilent 1100 series Liquid Chromatograph/Mass Selective Detector (MSD) (Single Quadropole) equipped with an electrospray interface and a UV diode array detector. Analyses were performed with methods using either an ACE 3 C8 (3.0 x 50 mm) column with a gradient of acetonitrile in 0.1% aqueous TFA over 3 min and a flow of 1 mL/min, or an Xbridge C18 (3.0 x 50 mm) column with a gradient of acetonitrile in 10 mM ammonium bicarbonate over 3 min and a flow of 1 mL/min.

Intermediate NMR spectra were recorded on a Nanalysis NMReady 60e at 25 °C. Final NMR spectra were recorded on a Bruker 400 MHz instrument at 25 °C °C using D6-DMSO as solvent and TMS as reference. Commercial names or trivial names were used for commercial materials and reagents.

### EXAMPLE 1

### Synthesis of Biotin-C10-ene

A: Synthesis of aminated biotin: N-(2-aminoethyl)-5-(2-oxo-1,3,3a,4,6,6a-hexahydrothieno[3,4-d]imidazol-4-yl)pentanamide.

Biotin was refluxed (5 gram, Aldrich) in methanol (100 ml) and TsOH (5 mg, Aldrich) overnight, then the reaction volume was reduced by gentle distillation in vacuum in rotary evaporator to produce a clear oil of the biotin methyl ester. Then 5 ml of 1,2 diamino ethane (Aldrich) in ethanol (25 ml) were added and the mixture was stirred overnight and then reduced by gentle distillation in vacuum in rotary evaporator to yield a light yellow semicrystalline oil. The aminated biotin was pure according to H-NMR, LC-MS and thin layer chromatography (TLC) and was used without further purification for the addition of VDMA (2-Vinyl-4,4-dimethyl azlactone, CAS No. 29513-26-6) in water in the next step.

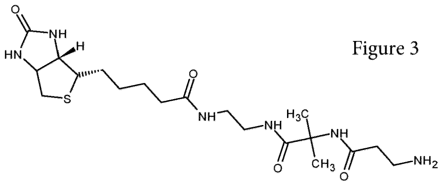

See Figure 3 illustrates position of the Biotin-linker (ethylendiamnie EDA, alpha-methyl alanine and beta-alanine)
B: To a solution of N-(2-aminoethyl)-5-(2-oxo-1,3,3a,4,6,6a-hexahydrothieno[3,4-d]imidazol-4- yl)pentanamide (1.0 g, 3.5 mmol) in water (20 mL) was added VDMA , 534 mg, 3.8 mmol). The reaction was stirred for 5 min and then freeze dried. The crude (1.56 g) was purified by silica chromatography column (80 g silica, liquid injection in methanol, DCM/MeOH, 5 % 2 CV, 5 to 20 % in 4 CV, 20 to 60 % in 2 CV, 60 % for 8 CV). The pooled fractions were concentrated. The residue was dissolved in water and freeze dried to yield **Biotin-C10-ene** as a white solid (1.2 g, 2.8 mmol, yield: 80 %). Mass spec.[M+H] + 426 Da.

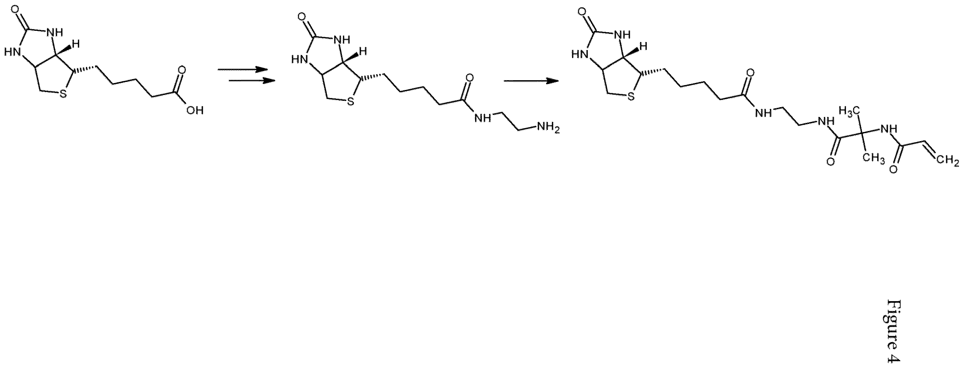

See Figure 4 illustrating the synthesis of **Biotin-C10-ene.**

The **Biotin-C10-ene** was used for the radical initiated co-grafting experiment on solid supports like membranes by co-polymerization with an acrylamide and could be used for coupling of the Biotin-C-10-ene to aminated substrates by Michael addition to the terminal vinyl bond.

### EXAMPLE 2

### Synthesis of Biotin-C10-NH₂

To Biotin-C10-ene (1.0 g, 2.3 mmol) was added a 12 N solution of ammonia in water (12 M, 23 mL, 282 mmol). The reaction mixture was stirred at 80 °C for 3 h in a closed pressure resistant vessel. The solution was freeze dried to obtain 1.0 g of Biotin-C10-NH₂. The compound was fully characterized by H-NMR, C-NMR and LCMS

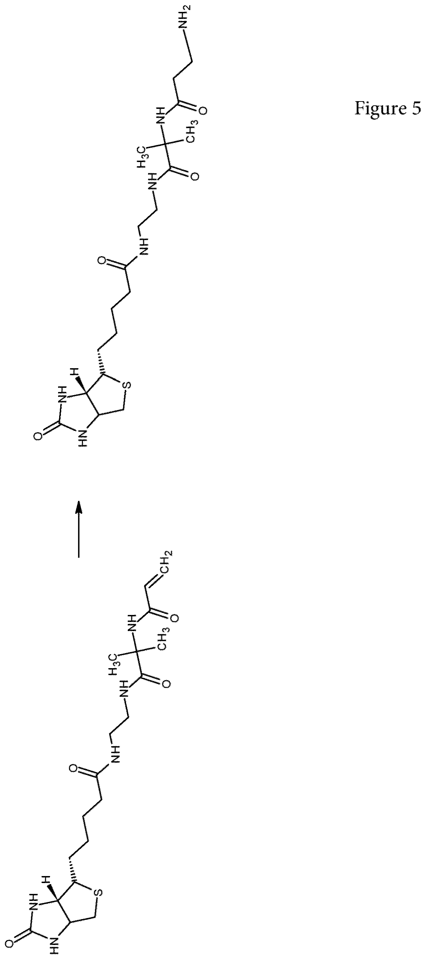

See Figure 5 illustrating the synthesis of Biotin-C10-NH₂.

### EXAMPLE 3

### Synthesis of Biotin-C17-ene

415 mg, (3.0 mmol) VMDA was added to a solution of Biotin-C10-NH2, synthesized as in example 2, (1.3 g, 2.9 mmol) in water (20 mL). The reaction was stirred for 5 min and then freeze dried. The crude solution (1.61 g) was purified by silica chromatography column (80 g silica column, liquid injection in methanol, DCM/MeOH 5 % 2 CV, 5 to 20 % in 3 CV, 20 to 60 % in 3 CV, 60 % 8 CV). The fractions containing the product and hydrolysed VDMA were pooled and concentrated by gentle distillation in vacuum in rotary evaporator. The material was further purified by basic alumina chromatography column (8 g alumina basic column, liquid injection in methanol, DCM/MeOH 0 to 5 % in 8 CV). The pooled fractions were concentrated by gentle distillation in vacuum in rotary evaporator. The residue was dissolved in water and freeze dried to yield Biotin-C17-ene as a white solid (425 mg, 0.73 mmol, yield: 32 %). Mass spec. [M+H] + 582 Da.

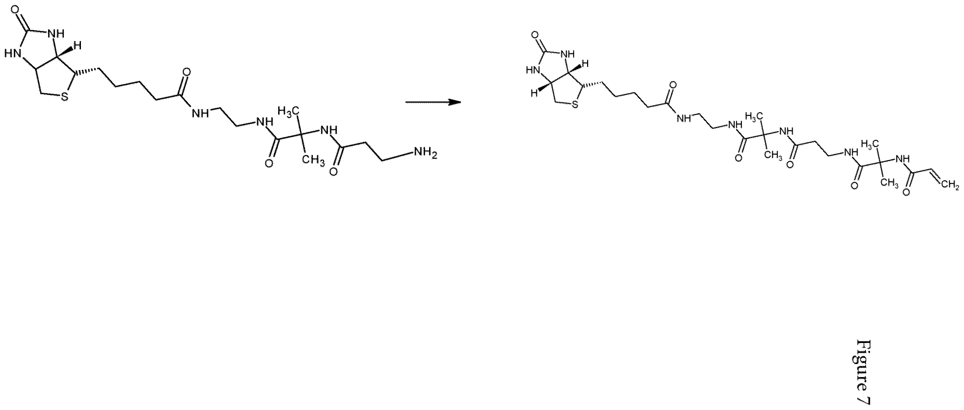

See Figure 7 illustrating the synthesis of **Biotin-C17-ene.**

**Biotin-C17-ene** was used for the radical initiated co-grafting experiment on membranes by co-polymerization with an acrylamide and used for coupling of the Biotin-C-17-ene to aminated substrates by Michael addition to the terminal vinyl double bond of Biotin-C17-ene

The linker was also used in a separate example for a further extension with another VDMA unit, creating an even longer linker derivative.

### EXAMPLE 4

This example illustrates the synthesis route for the Biotin-linkers of different lengths (C-10, C-17 and C-24). Two types of affinity ligands (series A: terminal NH₂ amino and series B: terminal vinyl -ene) with different linker length (C10, C17 and C24) were synthesized using peptide coupling reactions by HATU/DIPEA, and orthogonal N-terminal Fmoc and Boc deprotection by piperidine and 2 M HCl. The final steps are the N-terminal coupling of Biotin as active N-hydroxy succinimide (NHS) ester to the peptide linker molecules and final N-Boc deprotection for the synthesis of the N-terminal amino Biotin linker molecules Biotin-C10-NH₂, Biotin-C17-NH₂ and Biotin-C-24-NH₂ (series A).

The Biotin-linker molecules Biotin-C10-ene, Biotin-C17-ene, Biotin-C24-ene with terminal - ene groups (series B) have been synthesized by 2-Vinyl-4,4-dimethyl azlactone VDMA coupling onto the terminal amino group of Biotin-EDA-NH₂, Biotin-C10-NH₂ and Biotin-C17-NH₂ in water in order to achieve a C7 elongated linker molecule.

The first type, named Biotin-C10-NH₂, Biotin-C17-NH₂ Biotin-C24-NH₂, have a terminal amino group (series A). The second type, named Biotin-C10-ene, Biotin-C17-ene Biotin-C24-ene, have a terminal vinyl -ene group (series B).

### For each type below, three compounds were required with respectively n=1, 2 and 3.

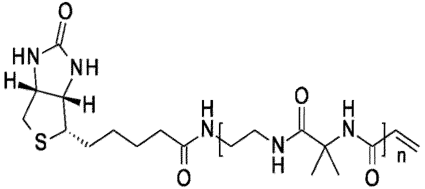

### Biotin-C10-ene, Biotin-C17-ene, Biotin-C24-ene .

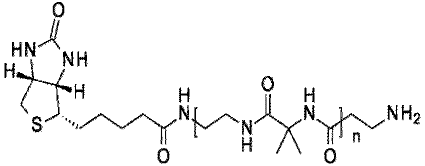

### Biotin-C10-NH₂, Biotin-C17-NH₂ and Biotin-C24-NH₂.

The approach used peptide coupling reactions. The chain of the linker is composed of three different components: Ethylene diamine EDA and the two amino acids: α-methyl alanine and β-alanine.

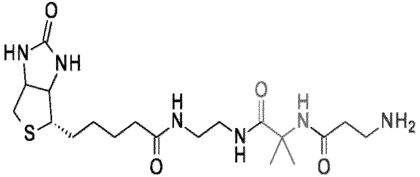

*The linker chain composed of ethylene diamine EDA, α-methyl alanine α-MeAla and β-alanine β-Ala.*

As an example, for the series B synthesis, shows Figure 7 adding 2-Vinyl-4,4-dimethyl azlactone VDMA to Biotin-C10-NH₂ will give the C7 elongated Biotin-C17-ene affinity ligand

### Synthesis of series A with terminal NH₂ (Biotin-C10-NH₂, Biotin-C17-NH₂ and Biotin-C24-NH₂)

### Synthesis of the different building blocks used in the synthesis of series A and series B linkers.

### Fmoc-EDA-HCl

To a solution of Fmoc-C! (16.1 g, 62.4 mmol) in dichloromethane (300 mL) cooled with ice bath, was added dropwise a solution of tert-butyl N-(2-aminoethyl) carbamate (Boc-EDA) (10.0 g, 62.4 mmol) in 20 mL DCM. The reaction was stirred at room temperature. After 30 min, a white precipitate formed. 10 mL of MeOH was added and the solution became homogenous. The mixture was washed with water (2 x 100 mL). The organic phase was dried over magnesium sulphate, filtered and concentrated. The solid was washed with diethyl ether and dried under vacuum to yield Fmoc-EDA-Boc (16.9 g, 44.2 mmol, yield: 70.8 %). To a solution of Fmoc-EDA-Boc (4.00 g, 10.5 mmol) in 1,4-dioxane (50.0 mL) was added a 4M solution of HCl in 1,4-dioxane (4.00 M, 55 mL, 220 mmol). The reaction mixture was stirred for 1 hour. The solid was filtered and washed with diethyl ether to yield Fmoc-EDA-HCl (3.5 g, 11.0 mmol, yield: quantitative) as a white solid.

### Biotin-NHS

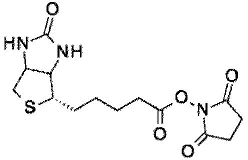

To a solution of N,N'-disuccinimidyl carbonate (12.7 g, 49.5 mmol) and biotin (11.0 g, 45.0 mmol) in N,N-dimethylformamide (50.0 mL) was added triethylamine TEA (31.4 mL, 0.225 mol). The reaction mixture was stirred for 16 hours. The solid was filtered, washed with diethyl ether and dried under vacuum to yield Biotin-NHS (11.2 g, 0.0328 mol, yield: 72.9 %) as a white solid and used without further purification.

### Fmoc-EDA-MeAla-NH₂-HCl and Fmoc-EDA-NH₂-HCl

To Fmoc-EDA-MeAla-NHBoc or Fmoc-EDA-NHBoc in 1,4-dioxane was added a 4 M solution of HCl in 1,4- dioxane (20 eq). The reaction mixture was stirred for 1 hour and then concentrated. The crude material was used without further purification.

### Procedure for Boc-α-methylalanine coupling

To a solution of Boc-o-methylalanine (1.2 eq) and HATU (1.1 eq) in acetonitrile (concentration ca 0.15 M) was added DIPEA (5 eq). The mixture was stirred for 10 min. To this mixture was added the *N*-terminal amino compounds (1 eq) and the resulting solution was stirred for 30 min. When the product precipitated, the solid was filtered, washed with diethyl ether and dried under vacuum. Otherwise, the solution was diluted with ethyl acetate, washed twice with a 0.5M solution of HCl in water, washed twice with water and washed once with brine. The organic phase was then dried over magnesium sulphate, filtered and concentrated.

### Procedure for Boc-β-alanine coupling

To a solution of Boc-*β*-alanine (1.2 eq) and HATU (1.1 eq) in acetonitrile (concentration ca 0.15 M) was added DIPEA (5 eq). The mixture was stirred for 10 min. To this mixture was added the *N*-terminal amino compound (1 eq) and the resulting solution was stirred for 30 min. When the product precipitated, the solid was filtered, washed with diethyl ether and dried under vacuum. Otherwise, the solution was diluted with ethyl acetate, washed twice with a 0.5M solution of HCl in water, washed twice with water and washed once with brine. The organic phase was then dried over magnesium sulphate, filtered and concentrated. In both cases, the crude material was used without further purification.

### NH₂-EDA-NHBoc

To Fmoc-ethylenediamine-NHBoc was added a 20 % solution of piperidine in acetonitrile. The reaction mixture was stirred for 30 minutes and then concentrated. The solution was concentrated, and the crude was dried 24 hours under vacuum. The solid was triturated three time with diethyl ether. The solid was filtered, washed with diethyl ether and dried under vacuum. The crude material was used without further purification.

### Biotin-EDA-NHBoc

To a solution of NH2-ethylenediamine-NHBoc in acetonitrile and water (concentration ca 0.1 M) was added Biotin-NHS (2 eq). The reaction mixture was stirred 30 min and check by LCMS. If needed additional 0.5 eq of Biotin-NHS was added and the mixture was stirred for 1 hour. The mixture was concentrated in rotary evaporator and purified by silica chromatography (liquid injection in methanol; DCM 2 CV, DCM/MeOH 0 to 60 % in 14 CV).

### Biotin-EDA-NH₂-HCl

To Biotin-Ethylenediamine-NHBoc was added a 2M solution of HCl in water. The reaction mixture was stirred overnight, and concentrated. The crude material was re-dissolved in methanol and concentrated. The solid was dried under vacuum.

### Synthesis of series A with terminal NH₂ (Biotin-C10-NH₂, Biotin-C17-NH₂ and Biotin-C24-NH₂)

### Biotin-C10-NH₂

Biotin-C10-NH₂ (Figure 5) has been synthesized by a series of peptide coupling reaction coupling reactions starting from HATU coupling of Boc-MeAla and N-Fmoc protected ethylene diamine Fmoc-EDA in acetonitrile to form FmocNH-EDA-MeAla-NHBoc. The terminal N-Boc protecting group was removed by 2 M HCl to for the N-terminal amino group Fmoc-EDA-MeAla-NH₂. The Fmoc-EDA-MeAla-NH₂ was coupled to the BocNH-β-alanine to form the Fmoc-EDA-MeAla-β-Ala-NHBoc. The terminal Fmoc group was removed by 20% piperidine in acetonitrile to form the terminal amino group. Then Biotin-NHS was coupled onto the NH₂-EDA-MeAla-β-Ala-NHBoc in acetonitrile-water at room temperature and finally the NHBoc group was removed with 2 M HCl at room temperature in order to synthesize the Biotin-C10-NH₂ with terminal amino group. The synthesis route is described in Figure 7. The compound Biotin-C10-NH₂ was fully characterized by H-NMR, C-NMR and LC-MS. Formula weight is 479.04, measured mass is found to be 478.21.

### Biotin-C17-NH₂

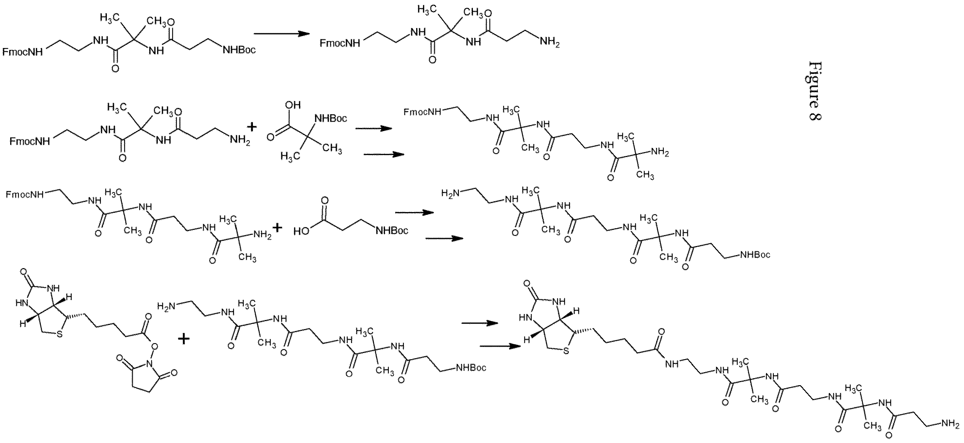

The synthesis of Biotin-C17-NH₂ (Figure 8) followed the same peptide coupling reactions by HATU/DIPEA, Boc deprotection with 2 M HCl, Fmoc-deprotection with piperidine and biotinylation with Biotin-NHS as described for Biotin-C10-NH₂

The Boc protecting group of Fmoc-EDA-MeAla-β-Ala-NHBoc (C-10 linker) was removed by 2 M HCl treatment to form Fmoc-EDA-MeAla-β-Ala-NH2. The terminal amino group was then coupled to Boc-MeAla by HATU/DIPEA in acetonitrile. The terminal Boc protecting group was removed by 2 M HCl and then coupled to β-Ala-NHBoc by HATU/DIPEA coupling. Then the Fmoc group was removed with 20% piperidine in acetonitrile, Biotin was coupled to the terminal NH2 group as NHS active ester and finally the terminal Boc group removed with 2 M HCl to form the Biotin-C17-NH₂ affinity linker molecule. Finally, after the deprotection of Biotin-C17-NHBoc, 1.5 g of product was obtained with an overall yield of 31 % (10 steps). The compound was fully characterized by H-NMR, C-NMR and LC-MS. Formula weight is 635.22, measured mass is found to be 634.31.

### Biotin-C24-NH₂

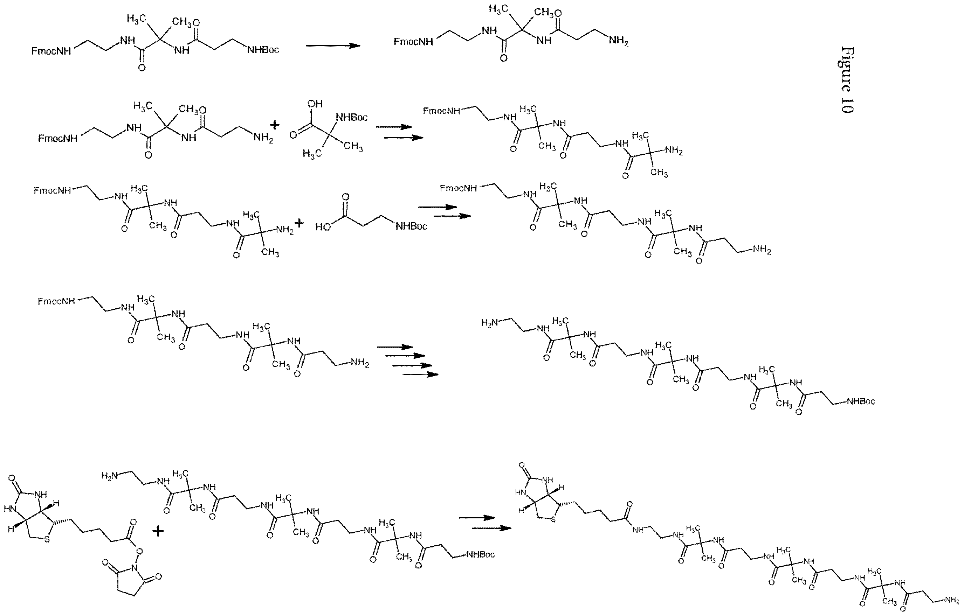

The synthesis of Biotin-C24-NH₂ (Figure 10) followed the same peptide coupling reactions by HATU/DIPEA, Boc deprotection with 2 M HCl, Fmoc-deprotection with piperidine and biotinylation with Biotin-NHS as described for Biotin-C10-NH₂ and Biotin-C17-NH₂.

The Boc protecting group of Fmoc-EDA-MeAla-β-Ala-MeAla- β-Ala-NHBoc (C-17 linker) was firstly removed by 2 M HCl treatment to form Fmoc-EDA-MeAla-β-Ala-MeAla- β-Ala-NH₂. The terminal amino group was then again coupled to Boc-MeAla by HATU/DIPEA in acetonitrile. The terminal Boc protecting group was again removed by 2 M HCl and then coupled to β-Ala-NHBoc by HATU/DIPEA coupling. Then the Fmoc group was removed with 20% piperidine in acetonitrile, Biotin was coupled to the terminal NH2 group as NHS active ester and finally the terminal Boc group removed with 2 M HCl to form the Biotin-C24-NH2 affinity linker molecule. With the experience from the synthesis of Biotin-C10-NH2 and Biotin-C17-NH₂, this synthesis of Biotin-C24-NH₂ resulted in 2 g of material were obtained with an overall yield of 30 % (14 steps). The compound was fully characterized by H-NMR, C-NMR and LC-MS. Formula weight is 791.40, measured mass is found to be 790.39.

### Synthesis of series B with terminal vinyl -ene (Biotin-C10-ene, Biotin-C17-ene and Biotin-C24-ene)

To a solution of Biotin-EDA-NH₂, Biotin-C10-NH₂ or Biotin-C17-NH₂ in water was added a 1 M solution of sodium bicarbonate in water until pH 8. To that solution was added VDMA. The mixture was concentrated and subjected to silica chromatography (liquid injection in methanol, DCM 2 CV, DCM/MeOH 0 to 20 % in 5 CV, 20 to 40 % in 3 CV, 40 to 60 % in 1 CV and 60 % for 3 CV). The pooled fractions were concentrated. The products were dissolved in methanol, concentrated and dried under vacuum to yield Biotin-C10-ene, Biotin-C17-ene and Biotin-C24-ene, respectively.

### Biotin-C10-ene

Biotin-C10-ene (Figure 4) with terminal vinyl group was synthesized by addition of 2-Vinyl-4,4-dimethyl azlactone VDMA to the terminal amino group of Biotin-EDA-NH2 in water. Biotin-C10-ene was finally purified by silica chromatography and fully characterized by H-NMR, C-NMR and LC-MS. Formula weight is 425.55, measured mass is found to be 425.21.

### Biotin-C17-ene

Biotin-C17-ene (Figure 7) with terminal vinyl group was accordingly synthesized by addition of 2-Vinyl-4,4-dimethyl azlactone VDMA to the terminal amino group of Biotin-C10-NH2 in water. Biotin-C17-ene was finally purified by silica chromatography and fully characterized by H-NMR, C-NMR and LC-MS. Formula weight is 581.73, measured mass is found to be 581.31

### Biotin-C24-ene

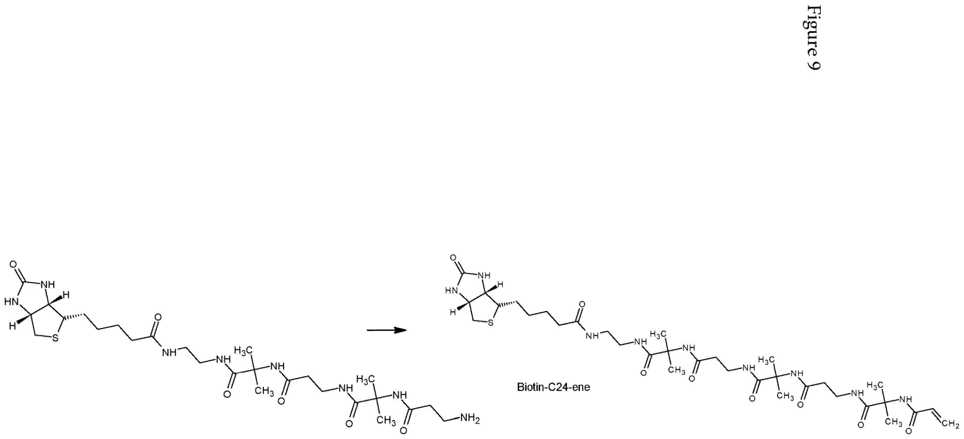

Biotin-C24-ene (Figure 9) with terminal vinyl group could be synthesized following the same strategy: Addition of 2-Vinyl-4,4-dimethyl azlactone VDMA to the terminal amino group of Biotin-C17-NH₂ in water resulted in the formation of the C7 elongated Biotin-C24-ene affinity ligand almost quantitatively. Biotin-C24-ene was finally purified by silica chromatography and fully characterized by H-NMR, C-NMR and LC-MS. Formula weight is 737.91, measured mass is found to be 737.39

### EXAMPLE 5

### Amination of bead substrate

WorkBeads 40/10 000 ACT (Bio-Works AB) containing reactive bromohydrin groups were treated with 1,2-diamino ethane (5% in water) at room temperature overnight. The beads were washed with water and a triethyl amine buffer (50 mM). The resulting primary amine functionalized beads were used for coupling of the medium and the long length linkers with biotin.

### EXAMPLE 6

### Coupling of Biotin-C10-ene or Biotin-C17-ene to aminated substrate:

The aminated substrate (example 5) was mixed with the Biotin-C10-ene or the Biotin-C17-ene synthesized as describes in example 1 and 3 respectively (50 nmol/ml aminated support) in 50 mM triethylamine (2ml/ml aminated support) for 6 hours. Reaction stopped by washed with water extensively to avoid any leakage.

The resulting two types of beads with **Biotin-C10** or **Biotin-C17,** may be used for purification of e.g. an antibody by capturing a Dual Affinity Polypeptide (DAP) or Target Binding Polypeptide (TBP) molecules according to previously described antibody purification methods, such as the purification processes disclosed in EP 2220107 B1 or in WO 2010/128033 A2.

### EXAMPLE 7

### Coupling of Biotin-C10-NH₂, Biotin-C17-NH₂ and Biotin-C24-NH₂ onto agarose resin with bromo-hydrine activation

- BioWorks Work Beads 40/10.000 ACT (4 ml, 150 µmol/ml bromo-hydrine activated, 50% slurry in 20% ethanol/water) were transferred to a glass filter, drained for 20% ethanol/water and washed several times with water and dried by suction for 5 min.
- Biotin-C10 (8,8 mg, 20 µmol), Biotin-C17 (12.0 mg, 20 µmol) and Biotin-C24 (15.1 mg, 20 µmol), were each dissolved in 4 ml 0,1 M Na₂CO₃/NaHCO₃ buffer, pH=9.3. The Biotin-C10-NH₂, Biotin-C17-NH₂ and Biotin-C24-NH₂ solution (4 ml) is then added to the Work Beads resin (4 ml) in a 10 ml plastic vial, respectively.
- The solution is then placed in a rotary mixer and agitated for 16 h at room temperature.
- The resins are then transferred to a glass filter, drained for the Biotin-C10-NH2, Biotin-C17-NH2 and Biotin-C24-NH2 solutions. The drained biotinylated WorkBeads are then washed with water (10x) and incubated with 5 ml of 1 M ethanol amine solution to inactivate the remaining bromo-hydrine activating groups on the agarose. The resins are then agitated in the rotary mixer for 8 h.
- Finally, the resins are transferred to a glass filter, drained for capping reagent 1M ethanol amine, washed with water (10 x), with 0.1 M HCL (5 x), and with water (10x), 20% ethanol (5x), dried by suction.
- The biotinylated resin preparations were stored in 20% ethanol as 50% slurry.

These biotinylated agarose resins with Biotin-C10-NH-, Biotin-C17-NH- and Biotin-C24-NH linker are tested for dynamic binding capacity and binding rate studies with DAP.

### EXAMPLE 8

### Measuring the Dynamic binding Capacity (DBC)

To determine the DBC of DAP or TBP to biotin linker coupled to the solid support surface the Langmuir adsorption isotherm is applied. This is based on the assumptions listed below:
- The surface is homogeneous.
- Adsorption cannot occur beyond monolayer coverage.
- All adsorption sites are equivalent and binding is independent of each other.
- There are no interactions between the adsorbed molecules and the molecules in the solution and no interactions between the solvent and the surface.

The Langmuir adsorption isotherm is based on the equilibrium between the DAP and the biotin linked to the solid support. Therefore, it is mandatory that DAP is in excess of the maximum binding capacity for each analysis to be able to reach equilibrium
Using the Langmuir isotherm, the binding of DAP to the biotin surface can be described as:

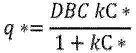

Which can be rearranged into:

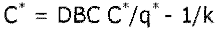

Where q* is the mass of bound DAP expressed as mg DAP bound per ml of biotin resin beads or membrane, c* is the equilibrium concentration of DAP in solution, DBC is the maximum binding capacity and k is the adsorption equilibrium constant. (ref: Beier et al, Adsorption of Amylase Enzyme on Ultrafiltration Membranes, Langmuir (2007), vol 23, pp 9341-51)

The DBC can be determined by plotting C* against C*/q*. The slope is equal to the DBC. Functionalized solid substrates prepared as described in the Examples above were analysed for dynamic binding capacity by incubating a known amount of solid substrate with four different amounts of DAP in solution.

The solid substrate (0.25 mL) was placed in an Omnifit column (6.6 mm/100 mm) (Diba Industries Inc., Danbury, CT, USA) and equilibrated with 0.3 M Citrate pH 3.5, 0.1% tween 20. An Äkta Pure (GE Healthcare, Chicago, IL, USA) was used to load 10 mL of DAP solution at concentrations of 1.8, 1.4, 1.1 and 0.9 mg/mL respectively. to the Omnifit column containing the functionalized solid substrate. The pump flow speed was set at 1.0 mL per minute, which corresponds to a residence time I the column of 15 seconds. After washing out unbound DAP, and collecting the flow through fraction, it was analysed for DAP content using a Nanodrop spectrometer (ThermoFisher, Waltham, MA, USA). Extinction coefficient for DAP: ε_{DAP} = 1.12 cm²/mg and MW=42,758 kDa

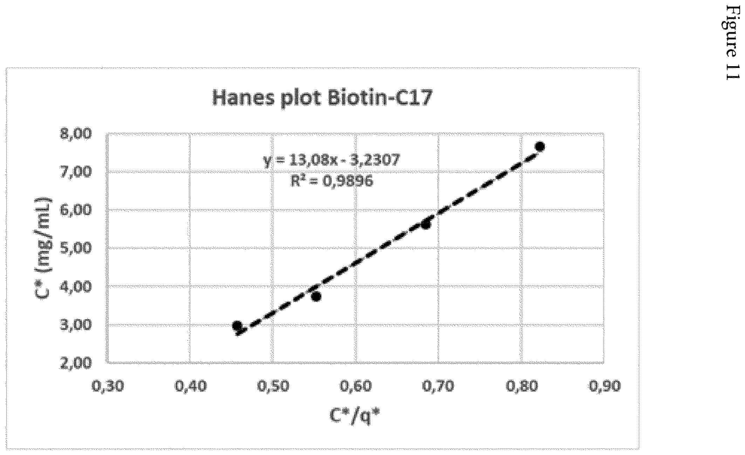

The equilibrium concentration in bulk liquid (C*, mg/mL) was calculated by determining the total DAP content in solution after incubation with the solid substrate. The equilibrium concentration (q*, mg/mL) on the solid substrate was calculated by subtracting the DAP content in

flow through solution from the starting DAP content in solution. The data collected from the experiments were used by plotting C* (y) as a linear function of C*/q* (x) as shown in Figure 11. The slope of the linear trendline gives the maximum binding capacity for the amount of solid substrate used in the binding experiment. In this analysis 0.25 mL of solid substrate was tested, to the dynamic binding capacity (DBC) in mg/mL is obtained by multiplying the slope by four.

In Table 1 are the DBC results summarised from analysing the Biotin-C10, Biotin-C17 andBiotin-C24 functionalized solid substrates. From the table it is clear that the Biotin-C17 functionalized solid substrate leads to the highest DBC.

**Table 1. Summary of determined dynamic binding capacity ("DBC", mg/mL) of the Biotin-C10, Biotin-C17 and Biotin-C24 functionalized solid substrate at a flow speed of 1.0 mL/minute, corresponding to a residence time of 15 seconds. A non-functionalized solid substrate was included as a control.**

| Compound | Coupling density | Number of spacer atoms | DBC (mg/ml) |
|---|---|---|---|
| Biotin-C10 | 5 µmol/ml | C10 | 35.9 |
| Biotin-C17 | 5 µmol/ml | C17 | 52.3 |
| Biotin-C24 | 5 µmol/ml | C24 | 33.1 |
| None | 0 µmol/ml | Null | 9.7 |

### EXAMPLE 9

### Binding rate (Residence time)

Functionalized solid substrates prepared as described in the Examples above were analysed further to understand if residence time will affect the DBC. For each pump speed was the DBC determined as above in example 8. Pump flow speed was varied to achieve different residence times of the DAP in contact with the solid substrate, i.e., 0.5, 1.0 and 2.0 mL per minute, which corresponds to residence times of 30, 15 and 7.5 seconds, respectively. The dynamic binding capacity was determined as described in EXAMPLE 9above for the Biotin-C10, Biotin-C17 and Biotin-C24 functionalized solid substrates and results are summarized in Table 2.

The Biotin-C17 functionalized solid substrate provide DBC maximum compared to the other Biotin Linkers. It is also observed that the DBC for Biotin-C17 is nearly constant (less than 4% reduction), which means the binding rate is much faster for Biotin-C17 compared to the other linker tested. Biotin-C10 and Biotin-C24 have DBC reduction of 20% and 10% respectively over the measured range of Residence times.

| Compound | Coupling density | Number of spacer atoms | Residence time (s) | DBC (mg/ml) |
|---|---|---|---|---|
| Biotin-C10 | 5 µmol/ml | C10 | 30 | 45,2 |
| Biotin-C10 | 5 µmol/ml | C10 | 15 | 43,3 |
| Biotin-C10 | 5 µmol/ml | C10 | 7,5 | 36,8 |
| Biotin-C17 | 5 µmol/ml | C17 | 30 | 50,0 |
| Biotin-C17 | 5 µmol/ml | C17 | 15 | 49,1 |
| Biotin-C17 | 5 µmol/ml | C17 | 7,5 | 47,9 |
| Biotin-C24 | 5 µmol/ml | C24 | 30 | 38,6 |
| Biotin-C24 | 5 µmol/ml | C24 | 15 | 34,6 |
| Biotin-C24 | 5 µmol/ml | C24 | 7,5 | 34,6 |

Table 2. Summary af measured dynamic binding capacity ("DBC", mg/mL) of the Biotin-C10, Biotin-C17 and Biotin-C24 functionalized solid substrate at flow speeds of 0.5, 1.0 and 2.0 mL/minute, corresponding to a residence time of 30, 15 and 7.5 seconds, respectively. The Biotin-C17 functionalized solid substrate leads to a higher DBC at all residence times measured and is affected only to a minor extent by the decrease in residence time.

### EXAMPLE 10

### Grafting Biotin-C10-ene, Biotin-C17-ene or Biotin-C24-ene onto membranes

Radical initiated co-grafting of three different lengths linkers: *Biotin-C10-ene, Biotin-Cl7-ene or Biotin-C24-ene,* respectively, with N,N-dimethyl acryl amide to polysulfone ether membrane sheets (3M).

The membrane was cut into round pieces (10 cm in diameter) and washed with water, followed by dioxane. The membrane was soaked in 10 % N,N-dimethyl acryl amide (Fluka) in dry and oxygen free dioxane and 0.1% of the Biotin-C10-ene, Biotin-C17-ene and Biotin-C24-ene, respectively, and with benzoequinone (Fluka, 25 ppm) as radical initiator. The membranes were irradiated with an UV lamp for 10 minutes under neutral atmosphere (dry nitrogen). The membranes were washed with dioxane.

Washed out homopolymer was collected and used to approximate the length of the grafted polymer by SEC. The homopolymer distribution was broad at approx. 500 to 25000 Da in molecular weight, as compared to a MW standard.

The resulting three different membranes with drafted poly acrylamide and **Biotin-C10, Biotin-C17** and **Biotin-C24** were washed extensively with hot water (60 °C) to remove any homopolymer and avoid later leakage.

The resulting membranes were physical intact, though slightly yellowish, and had graft copolymer consisting of a polyacrylamide backbone and the different lengths semi-stiff branches. The branches (Comb) different from the backbone.

The different membranes with polyacrylamide grafts with different lengths linkers from branch points (Comb) were tested in a set-up similar to example 8. The membranes was cut into small pieces (approx. 2x2 mm) and packed in the Omnifit column.

The experiment was repeated several times, due to problems with the folding of membranes in the column. The binding capacity was best for C17 (10-25 mg/mg membrane) about +15-25% better than the C10 and C24 preparations, which had almost the same capacity.

## Claims

1. A ligand-functionalized substrate comprising:
a. A substrate having carboxylic (-COOH), hydroxy (-OH), thio (-SH), amino groups (-NH₂), C-C double bonds (-ene) or C-C triple bonds (-yne) at the surface thereof, wherein the substrate is a solid substrate;
b. Covalently bound to said carboxylic (-COOH), hydroxy (-OH), thio (-SH), amino groups (-NH₂), C-C double bonds (-ene), C-C triple bonds (-yne) of said substrate a linker
and
c. A ligand-functional group
wherein said ligand-functional group and said linker are selected from the group consisting of: 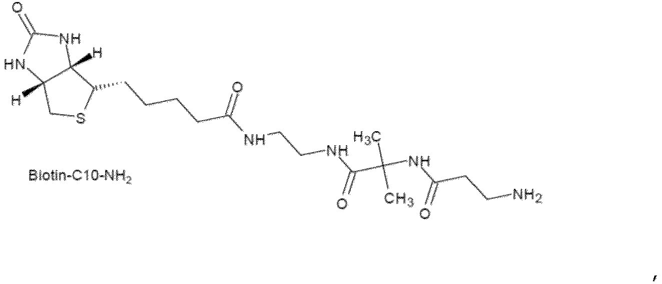 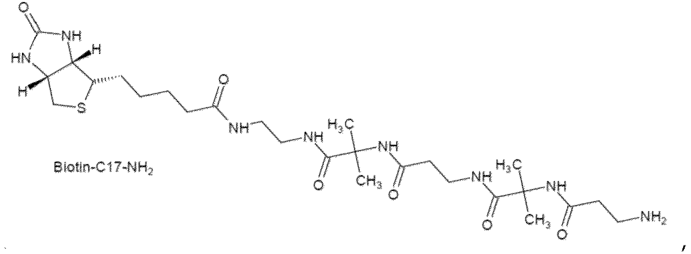 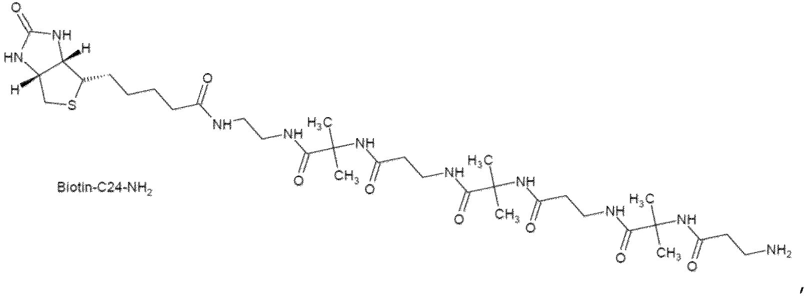 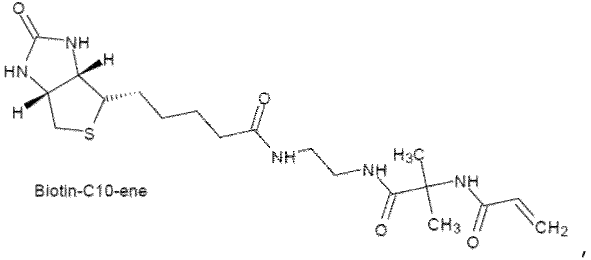 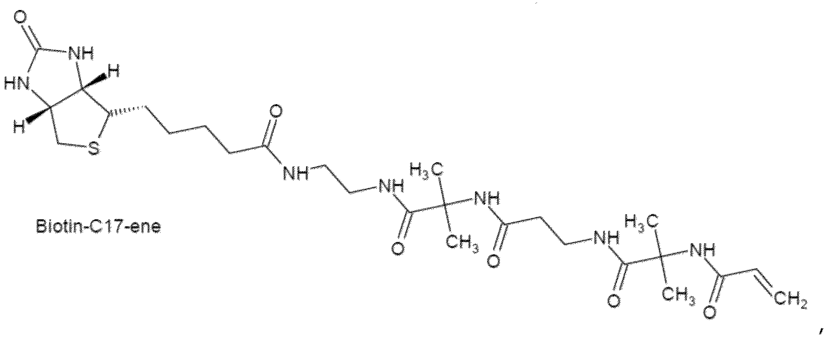 and

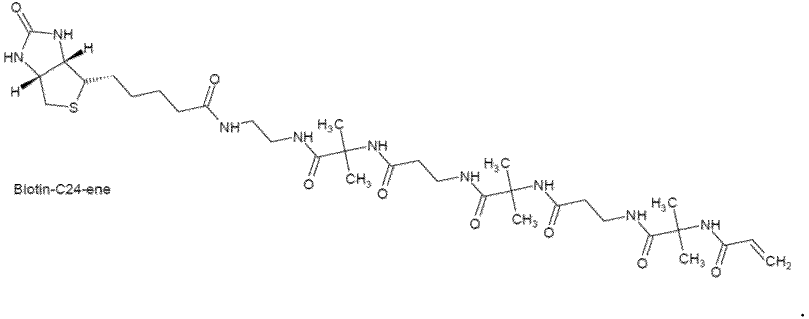

2. The ligand-functionalized substrate according to claim 1, wherein the substrate is grafted by radical initiated vinyl polymerization.

3. The ligand-functionalized substrate according to claim 1 or 2, wherein the substrate is selected from the group consisting of agarose, such as in the form of a resin or beads, kieselguhr, silica gel, cellulose, cellulose ethers, carboxymethyl cellulose, degenerated cellulose, agarose or paper based membranes, nitrocellulose, nitrocellulose mixed esters, silicas, and controlled pore glasses, polyamides, poly sulfone ether, polyvinylalcohols, polycarbonate, polyurethane, polyethersulfone, polysulfone, polyethylene terephthalate, polyvinylidene fluoride, polystyrene or polypropylene, polyethylene and co-block polymers, blends and combinations thereof.

4. The ligand-functionalized substrate according to any one of the preceding claims, wherein said ligand-functional group and said linker are selected from the group consisting of: 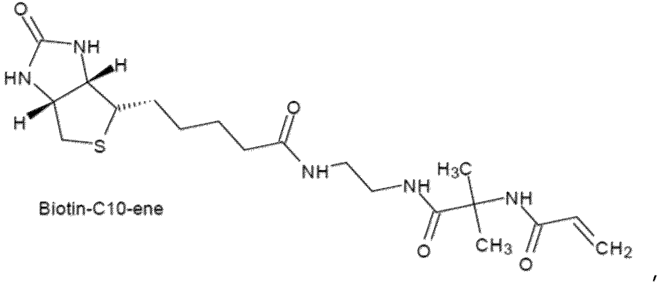 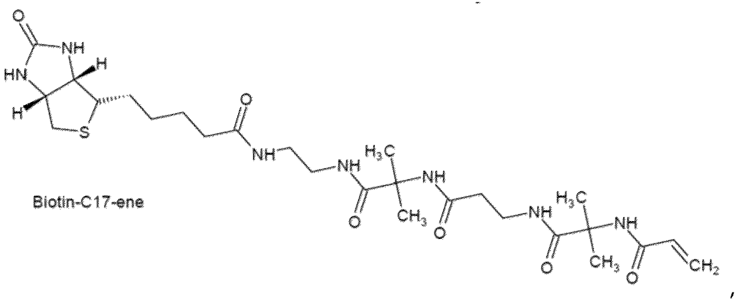 and

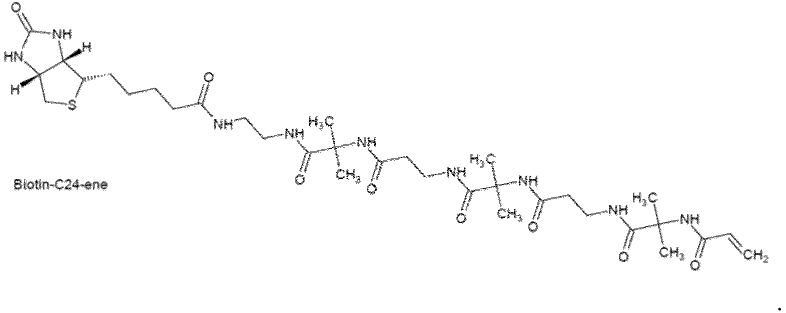

5. The ligand-functionalized substrate according to claim 4,
wherein the substrate is grafted by radical initiated vinyl polymerization.

6. The ligand-functionalized substrate according to claim 5, wherein the substrate is polysulfone ether.

7. A method for the preparation of the ligand-functionalized substrate as defined in any one of the preceding claims, comprising (a) activating the substrate chemically or by introducing a radical, and (b) coupling the ligand to the substrate directly via the linker or by radical co-polymerization using two or more acrylic monomers, of which at least one contains a ligand,
wherein the acrylic monomer that contains a ligand is selected from the group consisting of: 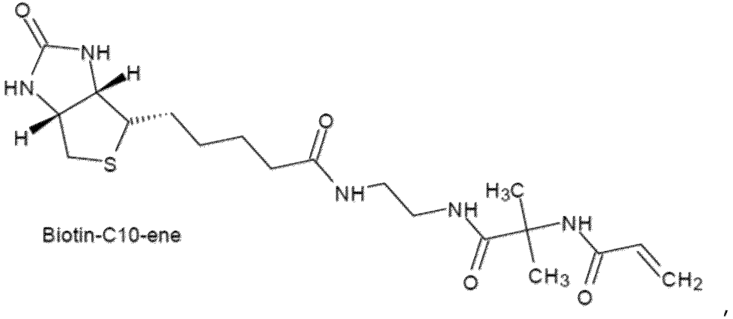 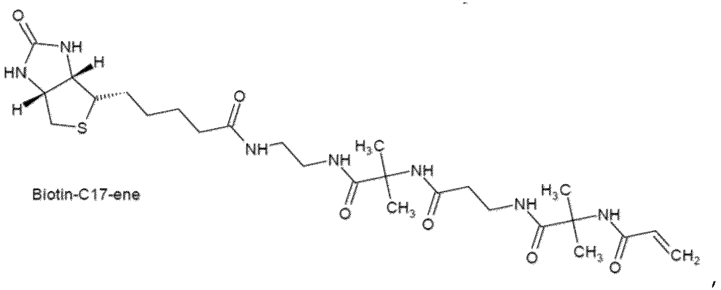 and

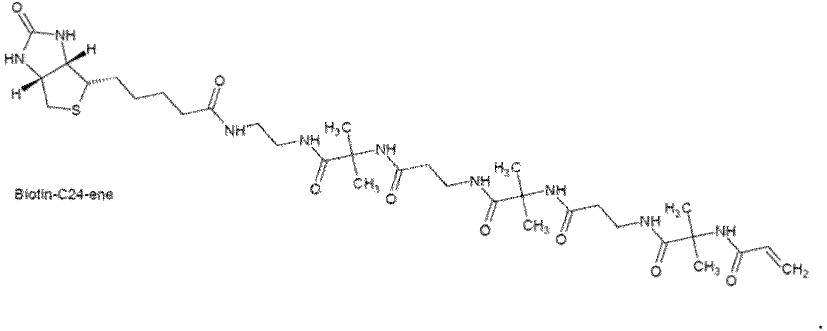

8. A use of a ligand-functionalized substrate according to any one of claims 1-7 for capturing or immobilizing a compound of interest.

## Patentansprüche

1. Ein Liganden-funktionalisiertes Substrat, umfassend:
a. Ein Substrat, das Carboxyl- (-COOH), Hydroxy- (-OH), Thio- (-SH), Aminogruppen (-NH₂), CC-Doppelbindungen (-en) oder CC-Dreifachbindungen (-in) an der Oberfläche davon aufweist, wobei das Substrat ein festes Substrat ist;
b. Kovalent gebunden an die Carboxyl- (-COOH), Hydroxy- (-OH), Thio- (-SH), Aminogruppen (-NH2), CC-Doppelbindungen (-en), CC-Dreifachbindungen (-in) des Substrats, einen Linker
und
c. Eine Liganden-funktionelle Gruppe
wobei die Liganden-funktionelle Gruppe und der Linker ausgewählt sind aus der Gruppe bestehend aus: 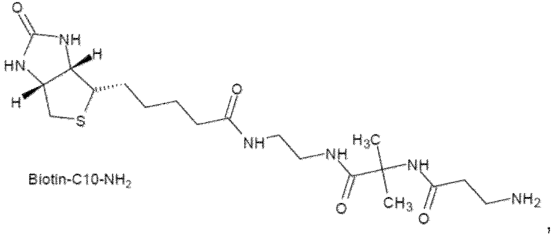 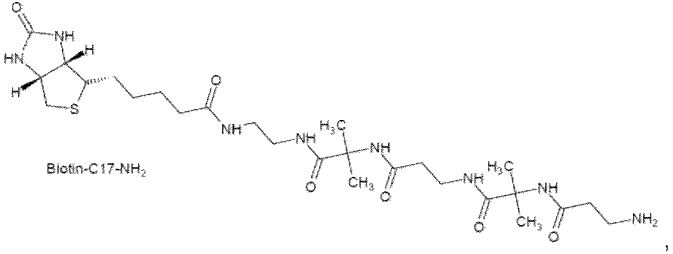 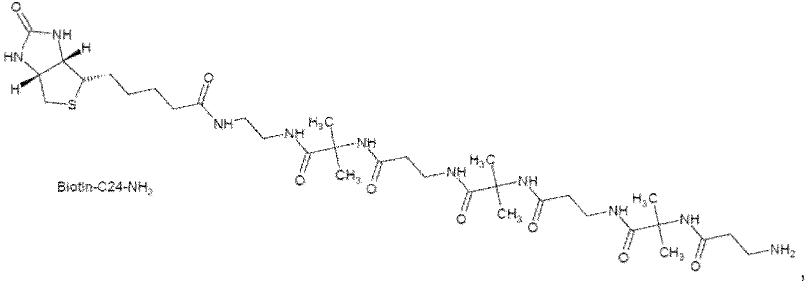 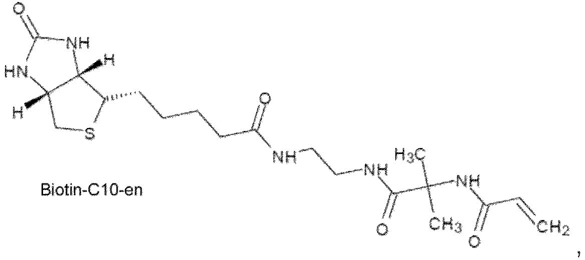 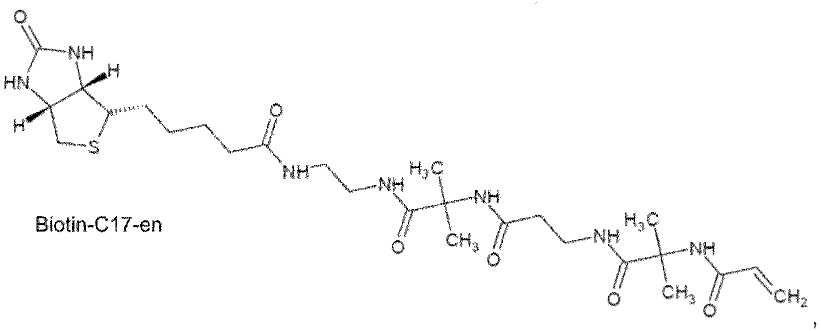 und

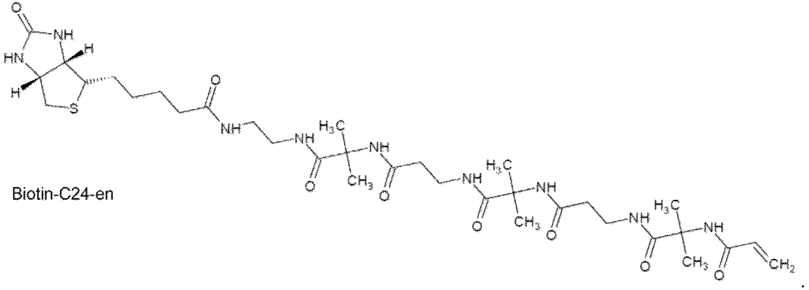

2. Das Liganden-funktionalisierte Substrat nach Anspruch 1, wobei das Substrat durch radikalisch initiierte Vinylpolymerisation gepfropft ist.

3. Das Liganden-funktionalisierte Substrat nach Anspruch 1 oder 2, wobei das Substrat ausgewählt ist aus der Gruppe bestehend aus Agarose, wie in Form eines Harzes oder von Perlen, Kieselgur, Kieselgel, Zellulose, Zelluloseethern, Carboxymethylzellulose, degenerierter Zellulose, Membranen basierend auf Agarose oder Papier, Nitrozellulose, Nitrozellulosemischester, Kieselsäuren und porenkontrollierten Gläsern, Polyamiden, Polysulfonethern, Polyvinylalkoholen, Polycarbonat, Polyurethan, Polyethersulfon, Polysulfon, Polyethylenterephthalat, Polyvinylidenfluorid, Polystyrol oder Polypropylen, Polyethylen und Coblockpolymeren, Mischungen und Kombinationen davon.

4. Das Liganden-funktionalisierte Substrat nach einem der vorstehenden Ansprüche, wobei die Liganden-funktionelle Gruppe und der Linker ausgewählt sind aus der Gruppe bestehend aus: 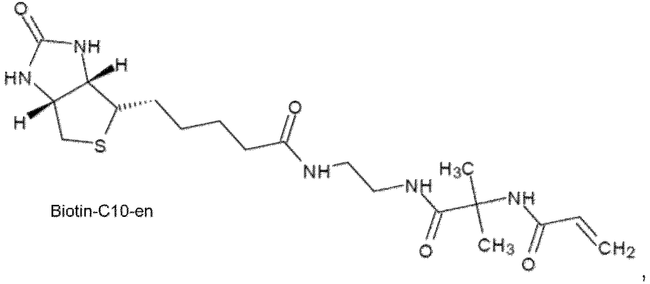 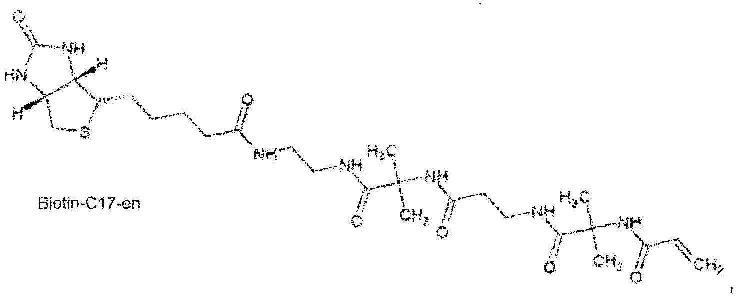 und

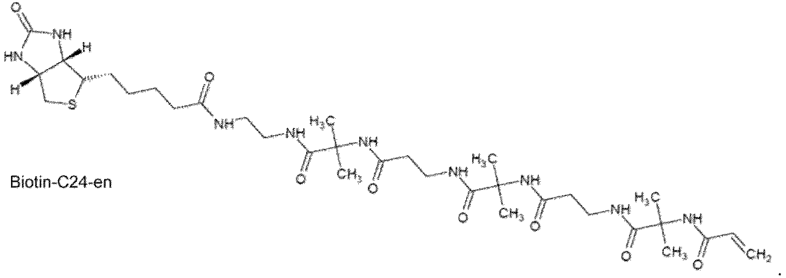

5. Das Liganden-funktionalisierte Substrat nach Anspruch 4, wobei das Substrat durch radikalisch initiierte Vinylpolymerisation gepfropft ist.

6. Das Liganden-funktionalisierte Substrat nach Anspruch 5, wobei das Substrat Polysulfonether ist.

7. Ein Verfahren für die Herstellung des Liganden-funktionalisierten Substrats nach einem der vorstehenden Ansprüche, umfassend (a) das chemische Aktivieren des Substrats oder durch Einführen eines Radikals und (b) das Koppeln des Liganden an das Substrat direkt über den Linker oder durch radikalische Copolymerisation unter Verwendung von zwei oder mehreren Acrylmonomeren, von denen mindestens eines einen Liganden enthält,
wobei das Acrylmonomer, das einen Liganden enthält, aus der Gruppe ausgewählt ist bestehend aus: 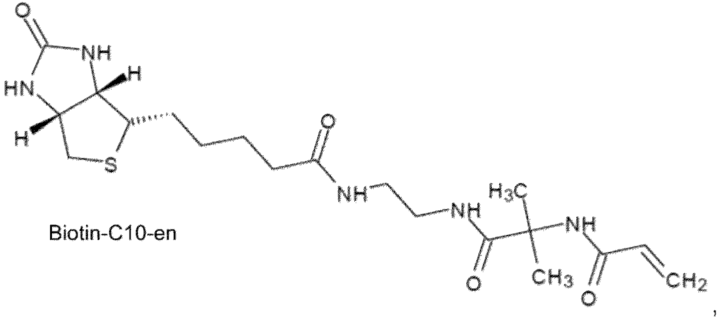 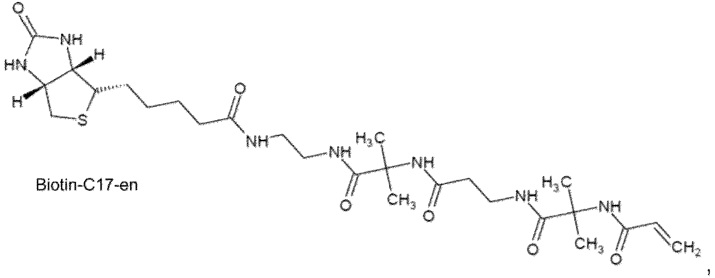 und

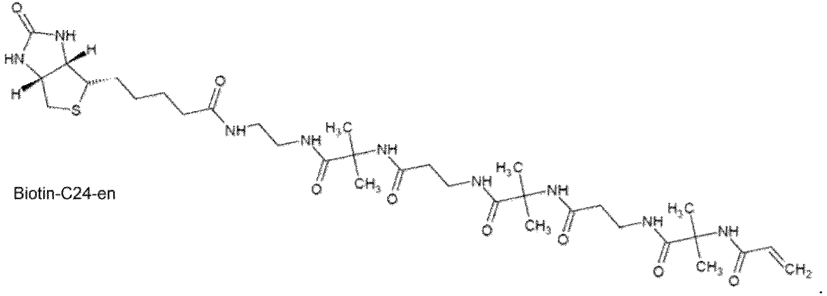

8. Eine Verwendung eines Liganden-funktionalisierten Substrats nach einem der Ansprüche 1 bis 7 zum Anlagern oder Immobilisieren einer Verbindung von Interesse.

## Revendications

1. Substrat fonctionnalisé par un ligand comprenant :
a. Un substrat ayant des groupes carboxyliques (-COOH), hydroxy (-OH), thio (-SH), amino (-NH₂), doubles liaisons C-C (-ène) ou triples liaisons C-C (-yne) à la surface de celui-ci, dans lequel le substrat est un substrat solide ;
b. Lié de manière covalente aux groupes carboxyliques (-COOH), hydroxy (-OH), thio (-SH), aminés (-NH2), doubles liaisons C-C (-ène), triples liaisons C-C (-yne) dudit substrat un agent de liaison
et
c. Un groupe fonctionnel ligand
dans lequel ledit groupe fonctionnel ligand et ledit agent de liaison sont choisis dans le groupe consistué de : 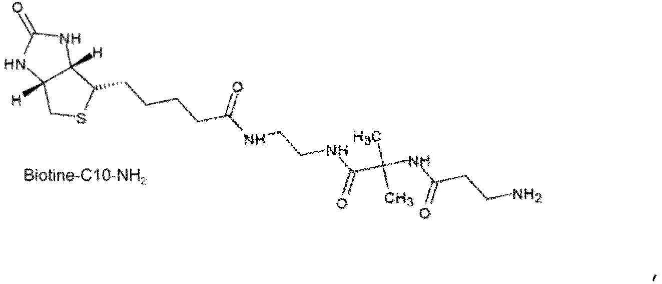 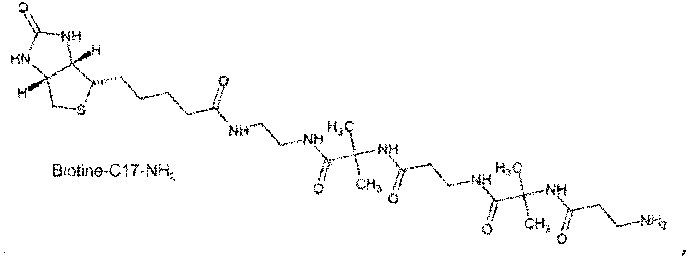 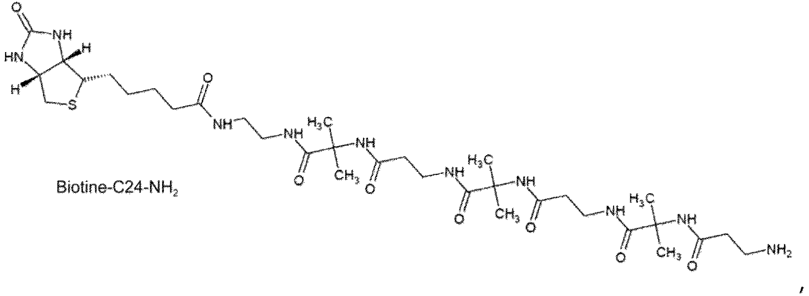 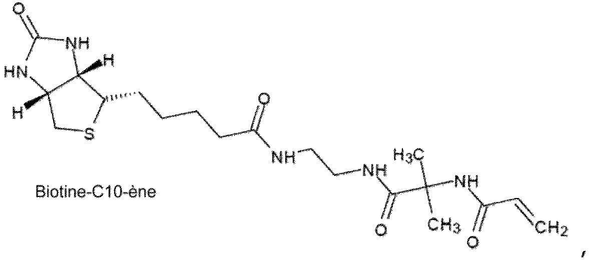 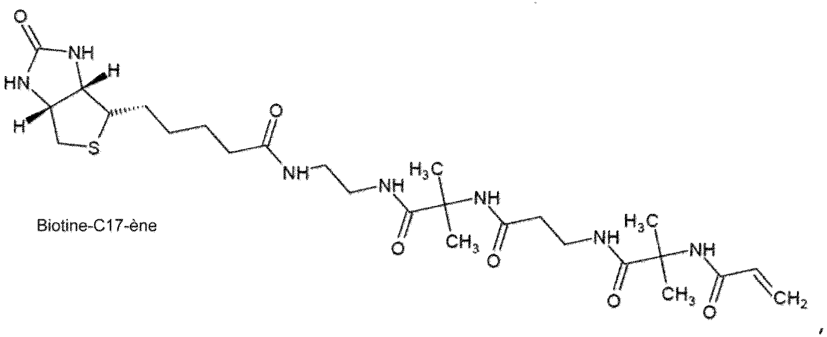 et

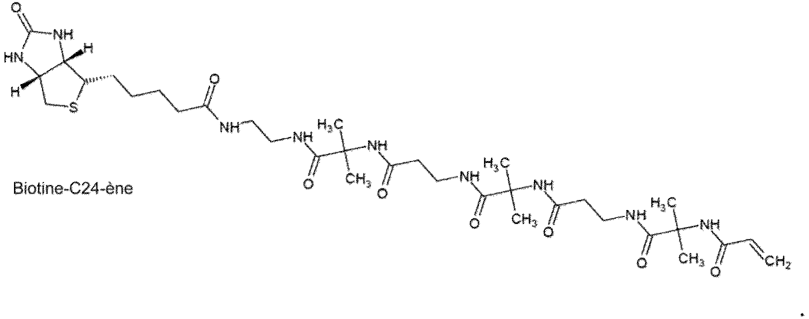

2. Substrat fonctionnalisé par un ligand selon la revendication 1, dans lequel le substrat est greffé par polymérisation vinylique initiée par un radical.

3. Substrat fonctionnalisé par un ligand selon la revendication 1 ou 2, dans lequel le substrat est choisi dans le groupe constitué d'agarose, sous forme de résine ou de billes, de kieselguhr, de gel de silice, de cellulose, d'éthers de cellulose, de carboxyméthylcellulose, de cellulose dégénérée, les membranes à base d'agarose ou de papier, la nitrocellulose, les esters mélangés de nitrocellulose, les silices et les verres à pores contrôlés, les polyamides, le polysulfone éther, les polyvinylalcohols, le polycarbonate, le polyuréthane, le polyéthersulfone, le polysulfone, le polyéthylène téréphtalate, le fluorure de polyvinylidène, polystyrène ou polypropylène, polyéthylène et polymères co-blocs, mélanges et combinaisons de ceux-ci.

4. Substrat fonctionnalisé par un ligand selon l'une quelconque des revendications précédentes, dans lequel ledit groupe fonctionnalisé par un ligand et ledit liant sont choisis dans le groupe constitué de : 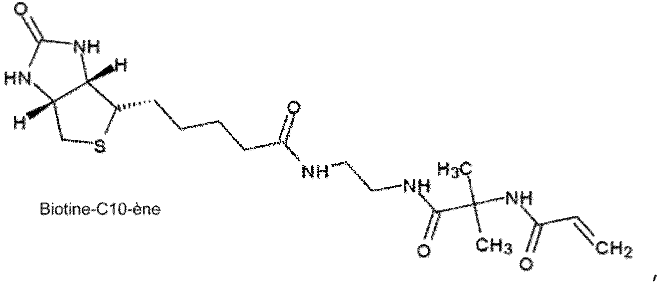 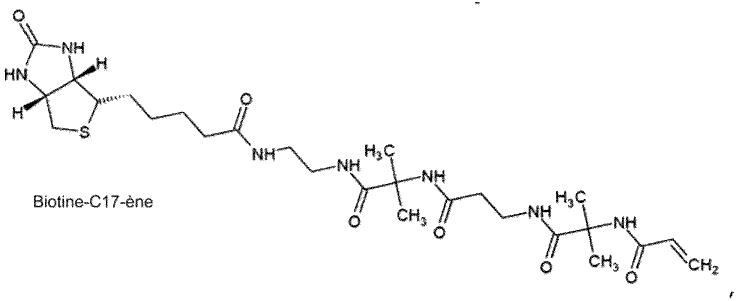 et

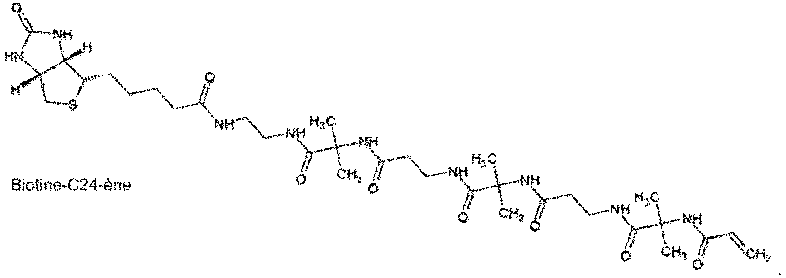

5. Substrat fonctionnalisé par un ligand selon la revendication 4, dans lequel le substrat est greffé par polymérisation vinylique initiée par un radical.

6. Substrat fonctionnalisé par un ligand selon la revendication 5, dans lequel le substrat est un éther de polysulfone.

7. Procédé de préparation du substrat fonctionnalisé par un ligand, tel que défini dans l'une quelconque des revendications précédentes, comprenant (a) l'activation chimique du substrat ou l'introduction d'un radical, et (b) l'accouplement du ligand au substrat directement par l'intermédiaire du linker ou par copolymérisation radicale à l'aide de deux monomères acryliques ou plus, dont l'un au moins contient un ligand,
dans lequel le monomère acrylique qui contient un ligand est choisi dans le groupe consistant de : 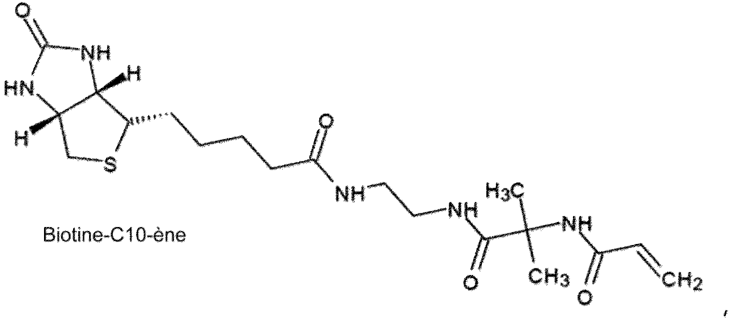 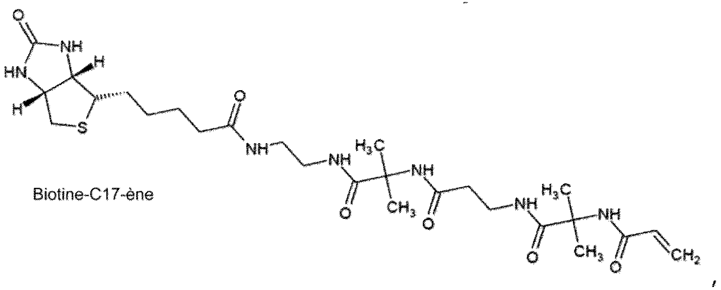 et

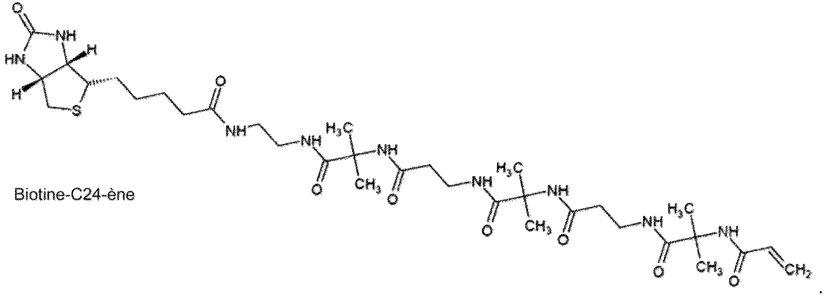

8. Utilisation d'un substrat fonctionnalisé par un ligand selon l'une des revendications 1 à 7 pour capturer ou immobiliser un composé d'intérêt.
